# EUROPEAN PATENT SPECIFICATION

(11) **EP 2 090 222 B1**
(45) Date of publication and mention of the grant of the patent: **16.03.2011**
(21) Application number: 08253984.2
(22) Date of filing: 12.12.2008
(51) Int. Cl.: A61B 3/135, A61B 3/117

(54) **Apparatus for cross-sectional imaging of anterior ocular segment**
Vorrichtung zur Querschnittsabbildung eines vorderen Augenelements
Appareil pour l'imagerie en coupe transversale d'un segment oculaire antérieur

(30) Priority: 14.02.2008 JP 2008032603
(43) Date of publication of application: 19.08.2009
(73) Proprietor: TOMEY CORPORATION, Nagoya-shi, Aichi 451-0051 (JP)
(72) Inventor: Nakashima, Masaya, Nagoya-shi Aichi 451-0051 (JP); Mizuno, Seinosuke, Nagoya-shi Aichi 451-0051 (JP)
(74) Representative: Jackson, Martin Peter

(56) References cited:
- EP-A- 0 933 060
- US-A- 4 711 541
- US-A1- 2007 182 927
- US-B1- 6 286 958

## Description

### INCORPORATED BY REFERENCE

The disclosure of Japanese Patent Application No. 2008-032603 filed on February 14, 2008 including the specification, drawings and abstract is incorporated herein by reference in its entirety.

### BACKGROUND OF THE INVENTION

### 1. Field of the Invention

The present invention relates to an apparatus for cross-sectional imaging of the anterior ocular segment, i.e. the anterior segment of the eye, wherein the device is adapted to take a cross-sectional image of the anterior ocular segment by directing an illuminating light source from the front into the examined eye, and detecting light scattered by the eye from the diagonal with respect to the eye.

### 2. Description of the Related Art

When screening for ocular disease, during diagnostic examination during refractive surgery of the eye or post-surgery, or when calculating the correct diopter for an intraocular lens, conventional practice is to examine and measure parameters such as the three-dimensional shape of the cornea, thickness of the cornea, and anterior chamber depth which represents the distance from the back face of the cornea to the front face of the crystalline lens.

There are a number of known anterior segment cross-sectional imaging apparatus adapted for non-contact cross-sectional imaging of the anterior segment of the examined eye when carrying out such examination and measurement of the cross-section of the anterior ocular segment. One of such anterior segment cross-sectional imaging apparatus, disclosed in Japanese unexamined Utility Model publication No. JP-U-50-143381, is adapted to image the anterior segment in cross section by directing a slit beam into the eye being examined, and using an imaging optical system situated on the diagonal with respect to the illuminating optical system, detect light scattered by the eye being examined. Most anterior segment cross-sectional imaging apparatus, like that disclosed in Japanese examined patent publication No. JP-B-59-27565, are designed to direct the slit beam from the front into the examined eye, and to allow the illuminating optical system and the imaging optical system to be rotated in unison about the eye-side optical axis of the illuminating optical system so as to be able to take a cross-sectional image of the anterior segment about the entire circumference.

However, unlike the case of a model eye made of glass or the like, when imaging the actual human eye, due to the presence of the nose there is always a risk of missing parts owing to the detected beam being partially obstructed by the nose. Thus, in order to avoid obstacles such as the nose so as to eliminate or reduce missing parts in the image, it will be preferable for the anterior segment cross-sectional imaging apparatus to be positioned as closely as possible to the examined eye during imaging. However, in a typical anterior segment cross-sectional imaging apparatus like that taught in JP-B-59-27565, since the illuminating optical system and the imaging optical system are designed to be rotated in front of the eye of the patient, the apparatus must be positioned some distance away so as to avoid hitting the patient's face, and particularly the nose. Another reason why imaging must be performed some distance away from the eye being examined is that the patient may experience some discomfort if the apparatus is positioned too close to the eye.

However, where the anterior segment cross-sectional imaging apparatus is positioned some distance away from the eye being examined, there is a significant problem of extraneous light (e.g. fluorescent light) entering the optical system, resulting in noise being introduced into the image. Preventing such infiltration of extraneous light required laborious measures such as carrying out imaging in a darkened room, using blackout curtains, and so on.

Owing to this inability to position the apparatus in sufficiently close proximity to the examined eye, it was necessary to employ lenses having sufficiently large diameter to take in the quantity of light needed for imaging. This fact, together with the fact that the imaging optical system is positioned on the diagonal with respect to the illuminating optical system, meant that such apparatus often tended to be very large. With a larger device, more time will be required to rotate the optical systems. Since the patient must keep his or her eyes open continuously during imaging, longer imaging times pose a considerable burden on the patient. Moreover, since images are taken from several directions, longer imaging times pose the problem that imaging cannot take place successfully due to tiny movements of the examined eye.
EP 0 933 060 A1 discloses a device according to the preamble of claim 1.

### SUMMARY OF THE INVENTION

It is therefore one object of the present invention to provide an apparatus of novel construction for cross-sectional imaging of the anterior ocular segment, that is adapted to be used in a lighted room and that affords more rapid imaging.

The above and/or optional objects of this invention may be attained according to at least one of the following modes of the invention. The following modes and/or elements employed in each mode of the invention may be adopted at any possible optional combinations.

A first mode of the present invention provides an apparatus for cross-sectional imaging of the anterior ocular segment, including: an illuminating optical system furnished with an illuminating light source for directing a slit beam from a front onto an examined eye; an imaging optical system furnished with a photoreceptor element that has an eye-side optical axis inclined by a prescribed angle with respect to an eye-side optical axis of the illuminating optical system and that detects scattered light produced by scattering of the slit beam by the examined eye and images a cross section of the examined eye; a stationary cylindrical body having apertures at both axial ends and being disposed projecting out from a body of the apparatus; the illuminating optical system and the imaging optical system being disposed within the cylindrical body with a front aperture of the cylindrical body as the common aperture thereof so as to be rotatable in unison about the eye-side optical axis of the illuminating optical system; and at least one proximal direction reflecting mirror being disposed within an interior of the cylindrical body in proximity to the front aperture for reflecting scattered light by the examined eye on an optical axis of the imaging optical system to a direction proximate to an optical axis of the illuminating optical system.

In the anterior segment cross-sectional imaging apparatus constructed according to the present invention, the optical axis of the imaging optical system will be reflected into proximity with the optical axis of the illuminating optical system by at least one proximal direction reflecting mirror which has been provided in the imaging optical system. The optical axis of the illuminating optical system and the optical axis of the imaging optical system will thereby be brought into proximity with each other so that the two optical systems can be disposed close to each other. As a result, it will be possible to achieve a more compact device.

Specifically, according to this anterior segment cross-sectional imaging apparatus, in order to obtain a cross-sectional image of the examined eye, the eye-side optical axis of the imaging optical system is given a prescribed inclination angle with respect to the eye-side optical axis of the illuminating optical system. Herein, an eye-side optical axis refers to an optical axis contiguous at one end to the examined eye. The eye-side optical axis of the illuminating optical system refers to the optical axis lying between the examined eye and the mirror situated furthest rearward in the direction of the optical axis going from the illuminant towards the examined eye. The eye-side optical axis of the imaging optical system refers to the optical axis lying between the examined eye and the reflecting mirror situated first in the direction of the optical axis going from the examined eye to the photoreceptor element. In a conventional anterior segment cross-sectional imaging apparatus, the direction of extension of the eye-side optical axis of the illuminating optical system and the direction of extension of the eye-side optical axis of the imaging optical system will gradually diverge moving away from the examined eye, with the apparatus as a whole tending to be larger in size as a result. According to the present invention however, by providing a proximal direction reflecting mirror for bringing the optical axis of the imaging optical system into proximity with the optical axis of the illuminating optical system, it will be possible to avoid positioning the optical axis of the imaging optical system away from the optical axis of the illuminating optical system, so that the illuminating optical system and the imaging optical system can be made more compact. In this mode in particular, since the proximal direction reflecting mirror is situated in proximity to the front aperture of the cylindrical body, the direction of the optical axis of the imaging optical system can be changed before there is appreciable divergence of the optical axis of the imaging optical system from the optical axis of the illuminating optical system, thus affording compact size more effectively.

Furthermore, according to this mode, because the illuminating optical system and the imaging optical system can be positioned at locations in proximity to one another, the illuminating optical system and the imaging optical system can be positioned inside the single cylindrical body so as to have the aperture thereof as their common aperture. The illuminating optical system and the imaging optical system will thereby be covered by the cylindrical body so that extraneous light can be blocked by the cylindrical body. As a result, it will be possible to carry out imaging in a lighted room without the need to use blackout curtains or darken the room. Furthermore, because the cylindrical body projects out from the body of the apparatus, the illuminating optical system and the imaging optical system can be positioned in closer proximity to the eye so that extraneous light can be prevented more effectively from entering. In particular, since the optical systems can be positioned in closer proximity to the eye beyond the patient's nose, the risk of missing parts of the cross-sectional image due to the light beam being obstructed by the nose can be reduced more effectively.

Additionally, because the imaging optical system can be positioned closer to the examined eye, the distance separating the eye and the lens can be made smaller. As a result, a sufficient quantity of light necessary for imaging can be assured with a lens of smaller diameter dimension, thus permitting the apparatus to be made even more compact.

In this way, according to the present invention, imaging can take place with the illuminating optical system and the imaging optical system positioned in closer proximity to the examined eye. Accordingly, while the illuminating optical system and the imaging optical system will need to be rotated at a location in proximity to the examined eye in order to image a cross-section of the anterior segment in the entire circumferential direction. In this mode, the two optical systems are rotated inside the cylindrical body while the cylindrical body itself is stationary and does not rotate. Thus, the patient is unlikely to become aware of the optical systems rotating just in front of the eye, so the patient will not experience any discomfort during imaging. Also, since the optical systems are covered by the cylindrical body, safety may be improved as well.

Also, owing to the more compact size of the rotating illuminating optical system and imaging optical system, the optical systems can rotate faster. As a result, the time required for imaging will be shorter, diminished imaging accuracy due to tiny movements of the examined eye will be reduced, and the burden on the patient to keep his or her eyes constantly open during the imaging procedure will be reduced.

A second mode of the present invention provides an apparatus for cross-sectional imaging of the anterior ocular segment according to the first mode, wherein the front aperture of the cylindrical body has a diameter dimension held within a range from 20mm to 60mm.

With the anterior segment cross-sectional imaging apparatus constructed according to this mode, it will be possible to effectively avoid contact of the cylindrical body against the nose of the patient, so that the cylindrical body, and hence the illuminating optical system and the imaging optical system housed within the cylindrical body, can be advantageously positioned closer to the examined eye.

A third mode of the present invention provides an apparatus for cross-sectional imaging of the anterior ocular segment according to the first or second mode, wherein the proximal direction reflecting mirror for reflecting the eye-side optical axis of the imaging optical system is disposed at location 40 mm or less from the front aperture of the cylindrical body in the direction of the eye-side optical axis of the illuminating optical system.

In the anterior segment cross-sectional imaging apparatus constructed according to this mode, the illuminating optical system and the imaging optical system may be more effectively made compact. Specifically, the distance separating the eye-side optical axis of the imaging optical system from the eye-side optical axis of the illuminating optical system becomes progressively greater further away from the front aperture of the cylindrical body. Therefore, if the proximal direction reflecting mirror for reflecting the eye-side optical axis of the imaging optical system is more than 40 mm away from the front aperture of the cylindrical body, the eye-side optical axes of the two optical systems will be too far apart, making it difficult to realize a compact design. In this mode, the location of the proximal direction reflecting mirror from the front aperture of the cylindrical body refers to the location from the front aperture of the cylindrical body, of the region at which the optical axis of the imaging optical system is reflected from the proximal direction reflecting mirror.

A fourth mode of the present invention provides an apparatus for cross-sectional imaging of the anterior ocular segment according to any one of the first to third modes, wherein an output optical axis of the illuminating optical system is orthogonal to the eye-side optical axis of the illuminating optical system.

In this mode, the output optical axis refers to an optical axis along which light emitted by the illuminant of the illuminating optical system travels in the optical axis direction to reach the first mirror. According to this mode, the dimension of the illuminating optical system in the direction of its eye-side optical axis can be limited so that the illuminating optical system can be more effectively made compact.

A fifth mode of the present invention provides an apparatus for cross-sectional imaging of the anterior ocular segment according to any one of the first to fourth modes, wherein a photoreception face of the photoreceptor element of the imaging optical system is oriented parallel to the eye-side optical axis of the illuminating optical system.

In the anterior segment cross-sectional imaging apparatus, the eye-side optical axis of the imaging optical system will be established on the diagonal with respect to anterior segment cross section (which is the observation plane). In order to achieve a focused image throughout the entire anterior segment cross section, the imaging optical system must be designed to fulfill the Scheimpflug condition. In order to fulfill the Scheimpflug condition in an illuminating optical system, a typical method is to incline the principal plane of the objective lens with respect to the optical axis of the imaging optical system, or to incline the photoreception face of the photoreceptor element with respect to the optical axis of the imaging optical system. In this mode, by employing the latter method while orienting the photoreception face of the photoreceptor element so as to lie parallel to the eye-side optical axis of the illuminating optical system, conditions are set so as to fulfill the Scheimpflug condition. By so doing, it is possible to reduce the size of the area occupied by the photoreceptor element in directions parallel and orthogonal to the direction of extension of the eye-side optical axis of the illuminating optical system, and to reduce the size of the area that is occupied by the photoreceptor element during rotation. A more compact size can be achieved thereby. In this mode, the term parallel is not used in a strict sense, and it is sufficient for the direction of extension of the photoreception face of the photoreceptor element to be approximately parallel to the eye-side optical axis of the illuminating optical system, to an extent such that the area occupied by the photoreceptor element during rotation can be reduced in size.

A sixth mode of the present invention provides an apparatus for cross-sectional imaging of the anterior ocular segment according to any one of the first to fifth modes, wherein the illuminating optical system, the imaging optical system, and the proximal direction reflecting mirror are situated inside an inner tube member that has been disposed inside the cylindrical body such that the illuminating optical system, the imaging optical system, and the proximal direction reflecting mirror are capable of rotation in unison with the inner tube member about the eye-side optical axis of the illuminating optical system; and the inner tube member is provided with a screening wall whereby an outside peripheral rim of the proximal direction reflecting mirror is not readily visible from an outside.

In the anterior segment cross-sectional imaging apparatus constructed according to this mode, the illuminating optical system, the imaging optical system, and the proximal direction reflecting mirror inside the inner tube member will not be readily visible to the patient as they rotate. The patient may thus have sense of assurance during the imaging process. In particular, according to this mode, because the outside peripheral rim (edge) of the proximal direction reflecting mirror is obscured by the screening wall, the patient have sense of assurance, and safety can be improved as well.

A seventh mode of the present invention provides an apparatus for cross-sectional imaging of the anterior ocular segment according to any one of the first to sixth modes, wherein the at least one proximal direction reflecting mirror comprises a plurality of proximal direction reflecting mirrors that are all situated inside the cylindrical body. In the anterior segment cross-sectional imaging apparatus constructed according to this mode, by situating all of the proximal direction reflecting mirrors inside the cylindrical body the proximal direction reflecting mirrors can be positioned stably at their relative locations, permitting more stable establishment of the direction of the optical axis of the imaging optical system.

An eighth mode of the present invention provides an apparatus for cross-sectional imaging of the anterior ocular segment according to any one of the first to seventh modes, wherein the at least one proximal direction reflecting mirror comprises a plurality of proximal direction reflecting mirrors that make up the illuminating optical system and the imaging optical system are situated within the cylindrical body as viewed in the axial direction of the cylindrical body; and the optical axis of the illuminating optical system and the optical axis of the imaging optical system extend in the axial direction of the cylindrical body within the cylindrical body as viewed in the axial direction of the cylindrical body. In this mode, the term reflecting mirrors refers not just to the proximal direction reflecting mirrors used to reflect scattered light from the examined eye in a direction in proximity to the optical axis of the illuminating optical system as taught in the first mode, and is used in a sense including the reflecting mirrors that make up the illuminating optical system and the imaging optical system as well. According to this mode, it will be possible to minimize the diameter dimension of rotating parts inclusive of the illuminating optical system and the imaging optical system, and to achieve production cost reductions as well.

Specifically, where for example a reflecting mirror is positioned at a location away from the cylindrical body as viewed in the axial direction of the cylindrical body so that the optical axis of the illuminating optical system and/or the optical axis of the imaging optical system extend out from the cylindrical body as viewed in the axial direction of the cylindrical body, the radius of rotation of the fastening members for fastening the reflecting mirrors, lenses, and other optical components that make up these optical systems will be larger than the radius of rotation of the cylindrical body. For this reason, rotating parts inclusive of the illuminating optical system and the imaging optical system will tend to be larger. Further, since the size of the cylindrical body on the patient-facing end thereof must be limited in terms of its diameter dimension so as to be able to be positioned in proximity to the examined eye while avoiding the nose, if optical components are situated at locations away from the cylindrical body as viewed in the axial direction it will be necessary for the fastening members to be larger only in those parts supporting these optical components. It is typical practice for fastening members used to fasten optical components to have cylindrical shape in order to prevent infiltration by extraneous light, but additional processes such as cutting will likely be needed in order to produce cylinders of different diameter dimension in the axial direction, with an associated risk of higher production costs. According to this mode, the diameter dimension of fastening members used to fasten optical components can be generally unchanging, and reduced production costs can be achieved.

Moreover, according to this mode, it is not necessary for the optical axes of the illuminating optical system and the imaging optical system to lie strictly parallel to the axial direction of the cylindrical body, and it is acceptable for the axes to extend on an incline to some degree with respect to the axial direction of the cylindrical body. Furthermore, it is not necessary for all of the plurality of reflecting mirrors that make up the illuminating optical system and the imaging optical system to be situated inside the cylindrical body as viewed in the axial direction of the cylindrical body, and it is acceptable for some of the plurality of reflecting mirrors to be situated within the cylindrical body as viewed in the axial direction, and for portions of the optical axes of the illuminating optical system and the imaging optical system to extend in the axial direction of the cylindrical body. In this case as well, it will be possible to reduce the diameter dimension of fastening members for fastening the reflecting mirrors that make up part of the optical axes, and for fastening the lenses situated on the optical axes.

A ninth mode of the present invention provides an apparatus for cross-sectional imaging of the anterior ocular segment according to any one of the first to eighth modes, further including XY direction position sensing means for sensing relative optical position of the illuminating optical system and the imaging optical system with respect to the examined eye in XY directions orthogonal to a Z direction which is a direction of moving closer to and away from the examined eye, as the illuminating optical system and the imaging optical system are moved in the XY directions, wherein the XY direction position sensing means is fixedly disposed such that an optical axis thereof and the eye-side optical axis of the illuminating optical system are identical.

In the anterior segment cross-sectional imaging apparatus constructed according to this mode, alignment of the illuminating optical system and the imaging optical system in the XY directions can be carried out by the XY direction position sensing means. Since the XY direction position sensing means is fixedly disposed and does not rotate, increased size of rotating parts can be avoided so as to overcome obstacles to making the illuminating optical system and the imaging optical system more compact. Thus, these optical systems can rotate faster. Furthermore, according to this mode, by situating the eye-side optical axis of the illuminating optical system (which is the center of rotation of the illuminating optical system and the imaging optical system) at a location identical to optical axis of the XY direction position sensing means and rotating the illuminating optical system and the imaging optical system about the optical axis of the XY direction position sensing means, a more compact arrangement for these optical systems will be possible. Additionally, where for example the anterior segment cross-sectional imaging apparatus of the present invention is furnished with a so-called topography optical system that projects a plurality of concentric rings onto the surface of the cornea and measures the shape of the cornea surface from the shapes of these multiple rings, and the XY direction position sensing means is switched between use in common with this topography optical system and with the illuminating optical system and imaging optical system in the present invention, by identically situating the center of rotation of the illuminating optical system and the imaging optical system with optical axis of the XY direction position sensing means, a switching structure for switching between the illuminating optical system and imaging optical system on the one hand and the topography optical system on the other can have a simpler design.

A tenth mode of the present invention provides an apparatus for cross-sectional imaging of the anterior ocular segment according to the ninth mode wherein the optical axis of the illuminating optical system and the optical axis of the imaging optical system extend in the same direction as the optical axis of the XY direction position sensing means at a location away from the eye-side optical axis of the illuminating optical system; and the optical axes of the illuminating optical system and the imaging optical system that extend in the same direction as the optical axis of the XY direction position sensing means are positioned at different circumferential locations about the optical axis of the XY direction position sensing means.

With this arrangement, the optical axes of the illuminating optical system and the imaging optical system can be brought closer to the optical axis of the XY direction position sensing means, allowing the rotating parts to be made more compact. Additionally, because the optical axes of the illuminating optical system and the imaging optical system rotate while the optical axis of the XY direction position sensing means is fixedly positioned, by disposing the illuminating optical system and the imaging optical system to either side of the optical axis of the XY direction position sensing means and rotating the illuminating optical system and the imaging optical system with the XY direction position sensing means as the center of rotation, a simpler and more compact design for the apparatus optical systems can be achieved.

Moreover, in this mode, it is more preferable that the optical axis of the illuminating optical system and the optical axis of the imaging optical system which extend in the same direction as the optical axis of the XY direction position sensing means will be situated to either side of the optical axis of the XY direction position sensing means, on the same given plane. By situating the illuminating optical system and the imaging optical system to opposite sides from one another with the optical axis of the XY direction position sensing means between them in this way, the illuminating optical system and the imaging optical system can be positioned with better balance in relation to the center of rotation, so that rotation stability may be improved. This advantageously ensures sufficient installation space for the parts that make up the illuminating optical system and the imaging optical system.

An eleventh mode of the present invention provides an apparatus for cross-sectional imaging of the anterior ocular segment according to any one of the first to tenth modes further including Z direction position sensing means for sensing the relative optical positions of the illuminating optical system and the imaging optical system in the Z direction with respect to the examined eye and adapted to rotate in unison with the illuminating optical system and the imaging optical system during movement of the illuminating optical system and the imaging optical system in the Z direction, which is the direction of moving closer to and away from the examined eye:

In the anterior segment cross-sectional imaging apparatus constructed according to this mode, alignment of the illuminating optical system and the imaging optical system in the Z direction can be carried out by the Z direction position sensing means. Since the Z direction position sensing means is adapted to rotate in unison with the illuminating optical system and the imaging optical system, a compact arrangement for the Z direction position sensing means is possible. In this mode in particular, a mode wherein the optical axis of the Z direction position sensing means is partially common to the optical axis of the imaging optical system is preferably employed. A more compact arrangement for the Z direction position sensing means is possible by employing components of the imaging optical system in common as optical components of the Z direction position sensing means.

### BRIEF DESCRIPTION OF THE DRAWINGS

The foregoing and/or other objects features and advantages of the invention will become more apparent from the following description of a preferred embodiment with reference to the accompanying drawings in which like reference numerals designate like elements and wherein:
FIG. 1 is a schematic view for explaining an optical system of an apparatus for cross-sectional imaging of the anterior ocular segment according to one embodiment of the present invention;
FIG. 2 is a longitudinal cross sectional view illustrating a primary part of the apparatus for cross-sectional imaging of the anterior ocular segment having the optical system of FIG. 1;
FIG. 3 is a schematic view for explaining a method of alignment in Z direction;
FIG. 4 is a graph for explaining photoreception signals detected by a Z alignment sensor;
FIG. 5 is a schematic view for explaining a compactness of the apparatus as one effect of the present invention;
FIG. 6 is a schematic view for explaining the difference in size between the conventional structure and the present invention; and
FIG. 7 is a schematic view for explaining one modification of the present invention.

### DETAILED DESCRIPTION OF PREFERRED EMBODIMENTS

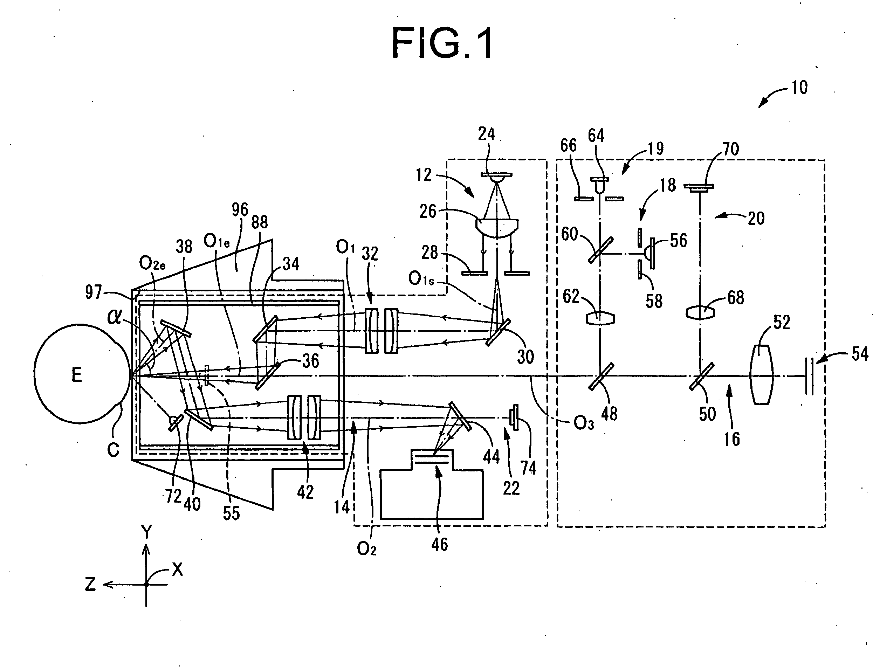

First, FIG. 1 depicts an apparatus optical system 10 according to a first embodiment of an apparatus for cross-sectional imaging of an anterior ocular segment of the present invention. The apparatus optical system 10 includes an illuminating optical system 12 for directing a slit beam onto an examined eye E; an imaging optical system 14 that serves as an imaging optical system for detecting light of the slit beam that was directed by the illuminating optical system 12 onto the examined eye E and scattered thereby, and for imaging a cross section of the anterior segment of the examined eye E; an observation optical system 16 for observing the anterior segment of the examined eye E; a fixation target optical system 18 for producing a fixation target light; an XY alignment optical system 19 and an XY alignment detection optical system 20 serving as XY direction position sensing means for positioning the apparatus optical system 10 in the XY direction (in FIG. 1, the direction perpendicular to the plane of the page is the X direction and the vertical direction in the plane of the page is the Y direction) orthogonal to the Z direction which is the direction moving closer to or away from the examined eye E (in FIG. 1, the left-right direction in the plane of the page); and a Z alignment optical system 22 serving as Z direction position sensing means for positioning the apparatus optical system 10 in the Z direction. Unless indicated otherwise, frontward is used herein to refer to the leftward direction in FIG. 1, and the vertical direction to refer to the vertical direction in FIG. 1.

Turing now to a more detailed description, the illuminating optical system 12 is composed, in order from the location furthest away from the examined eye E, of an illuminant light source 24; a projector lens 26, a slit 28, a mirror 30, a projector lens 32, a mirror 34, and a dichroic mirror 36, arranged on an optical axis O₁. The illuminant light source 24 employs a LED that emits a beam of visible light, for example. The dichroic mirror 36 reflects a certain wavelength range of the light from the illuminant light source 24, while transmitting light of other wavelength ranges.

The beam of light emitted from the illuminant light source 24 is collimated into a parallel beam by the projector lens 26, and after passing through the slit 28 emerges as a slit beam which is reflected from the mirror 30. In this embodiment, the optical axis along which the light emitted from the illuminant light source 24 travels until reflected by the mirror 30 is designated as the output optical axis O₁ₛ of the illuminating optical system 12. In this embodiment in particular, the illuminant light source 24, the projector lens 26, and the slit 28 are arranged in a direction orthogonal to the Z direction, and the direction of the output optical axis O₁ₛ is orthogonal to the Z direction.

Next, the beam that was reflected from the mirror 30 approaches the examined eye E in a direction substantially parallel to the Z direction, and after passing through the projector lens 32 is reflected from the mirror 34 in a direction orthogonal to the Z direction. The beam that was reflected by the mirror 34 is then reflected by the dichroic mirror 36, and thereby directed from the front onto the cornea C of the examined eye E in a direction parallel to the Z direction. Thus, in this embodiment, the optical axis traveled by light reflected by the dichroic mirror 36 to the examined eye E is designated as the eye-side optical axis O₁ₑ of the illuminating optical system 12.

Thus, in this embodiment, on the optical axis O₁ of the illuminating optical system 12, the direction of the output optical axis O₁ₛ will be orthogonal to the direction of the eye-side optical axis O₁ₑ. Also, on the optical axis O₁, the optical axis segment between the mirror 30 and the mirror 34 will be established so as to extend in the Z direction, away from an extended line of the eye-side optical axis O₁ₑ.

Meanwhile, the imaging optical system 14 is composed, in order from the location closest to the examined eye E, of a mirror 38, a mirror 40, an objective lens 42, a dichroic mirror 44, and a CCD 46 (photoreceptor element), arranged on an optical axis O₂. The dichroic mirror 44 reflects a certain wavelength range of the light that has been directed from the illuminant light source 24 onto and scattered by the cornea C of the examined eye E, while transmitting light of other wavelength ranges. The beam of light that was emitted by the illuminant light source 24 and directed through the illuminating optical system 12 onto the cornea C of the examined eye E and scattered thereby is then reflected by the mirror 38 and the mirror 40, and after emerging in the Z direction at a location away from the eye-side optical axis O₁ₑ of the illuminating optical system 12, is reflected by the dichroic mirror 44 via the objective lens 42, and is focused onto the CCD 46.

In order to image a cross section of the anterior segment of the examined eye E inclusive of the cornea C and the crystalline lens (not shown), the imaging optical system 14 receives scattered light from the examined eye E on the diagonal. Consequently, the eye-side optical axis O₂ₑ extending from the examined eye E to the mirror 38 on the optical axis O₂ of the imaging optical system 14 will be inclined by a prescribed angle: α with respect to the eye-side optical axis O₁ₑ of the illuminating optical system 12. The inclination angle: α of the eye-side optical axis O₂ₑ with respect to the eye-side optical axis O₁ₑ will lie in a range of 30 - 60 degrees, preferably 40 - 50 degrees, and more preferably 43 - 47 degrees. In this embodiment in particular, the inclination angle: α has been set to 45 degrees. That is, if the inclination angle: α is greater than 60 degrees, it will not be possible to receive scattered light unless positioned excessively close to the examined eye E, thus making it difficult to receive scattered light; whereas if the inclination angle: α is less than 30 degrees, the scattered light will be received generally from the front, and the cross section obtained thereby will be thin.

In this embodiment, the eye-side optical axis O₂ₑ of the imaging optical system 14 is established on the diagonal with respect to the observation plane (the anterior segment cross section). Therefore, in order to obtain an in-focus image of the anterior segment cross section in its entirety, the principal plane of the objective lens 42 and the photoreception face of the CCD 46 of the imaging optical system 14 must have inclination angles with respect to the optical axis O₂ₑ of the imaging optical system 14 that have been established so as to fulfill the Scheimpflug condition. In this embodiment in particular, the optical axis O₂ of the imaging optical system 14 is made parallel to the eye-side optical axis O₁ₑ of the illuminating optical system 12 by the mirrors 38, 40, whereby the objective lens 42 is oriented with its principal plane orthogonal to the eye-side optical axis O₁ₑ; and the optical axis O₂ is reflected by the dichroic mirror 44 so as to impinge on the CCD 46 on the diagonal, whereby the photoreception face of the CCD 46 is oriented parallel to the eye-side optical axis O₁ₑ. Thus, during rotation of the illuminating optical system 12 about the eye-side optical axis O₁ₑ (discussed later), the photoreception face of the CCD 46 will occupy a smaller area as compared to the case where the photoreception face of the CCD 46 is positioned on the diagonal with respect to the eye-side optical axis O₁ₑ.

The observation optical system 16 is composed, in order from the location closest to the examined eye E, of the dichroic mirror 36, a dichroic mirror 48, a dichroic mirror 50, an objective lens 52, and a CCD 54, arranged on an optical axis O₃. The dichroic mirrors 48, 50 transmit the wavelength region of light that has been directed onto the examined eye E by an observation light source 55 situated in proximity to the examined eye E and subsequently scattered by the anterior ocular segment, while reflecting the remaining wavelength regions. In this embodiment, the observation light source 55 employs an LED that emits infrared light. The observation light source 55 is situated at a location away from the eye-side optical axis O₁ₑ of the illuminating optical system 12 and slightly to the rear of the mirror 38 in the direction of the eye-side optical axis O₁ₑ. The optical axis O₃ of the observation optical system 16 is coincident with the eye-side optical axis O₁ₑ of the illuminating optical system 12. The beam emitted by the observation light source 55 and subsequently scattered by the anterior segment of the examined eye E will pass through the dichroic mirrors 36, 48, 50 and the objective lens 52, becoming focused on the CCD 54.

The fixation target optical system 18 is composed, in order from the location furthest away from the examined eye E, of a fixation target light source 56, a pinhole plate 58, a dichroic mirror 60, a projector lens 62, the dichroic mirror 48, and the dichroic mirror 36. The optical axis of the fixation target optical system 18 is partially coincident with the eye-side optical axis O₁ₑ of the illuminating optical system 12. The dichroic mirror 60 reflects the wavelength region of light from the fixation target light source 56, while transmitting other wavelength regions. The fixation target light source 56 is a light source that emits visible light, such as an LED for example. The beam emitted by the fixation target light scurce 56 passes through the pinhole plate 58 and is reflected from the dichroic mirror 60, then collimated into a parallel beam by the projector lens 62, reflected from the dichroic mirror 48, and transmitted through the dichroic mirror 36 to impinge on the examined eye E.

The XY alignment optical system 19 is composed, in order from the location furthest away from the examined eye E, of an XY alignment light source 64, a pinhole plate 66, the dichroic mirror 60, the projector lens 62, the dichroic mirror 48, and the dichroic mirror 36. The optical axis of the XY alignment optical system 19 is partially coincident with the eye-side optical axis O₁ₑ of the illuminating optical system 12. The beam emitted by the XY alignment light source 64 passes through the pinhole plate 66 and the dichroic mirror 60, is then collimated into a parallel beam by the projector lens 62, reflected from the dichroic mirror 48, and transmitted through the dichroic mirror 36 to impinge on the examined eye E.

The XY alignment detection optical system 20 is composed, in order from the location closest to the examined eye E, of the dichroic mirror 36, the dichroic mirror 48, the dichroic mirror 50, an objective lens 68, and an XY alignment sensor (profile sensor) 70 capable of sensing the position of the incident beam. The optical axis of the XY alignment detection optical system 20 is partially coincident with the optical axis of the XY alignment optical system 19, and coincident with the eye-side optical axis O₁ₑ of the illuminating optical system 12. The beam of light directed onto the examined eye E from the XY alignment light source 64 and reflected by the anterior ocular segment is reflected by the dichroic mirror 50 on the optical axis O₃ of the observation optical system 16, and guided to the XY alignment sensor 70 via the objective lens 68.

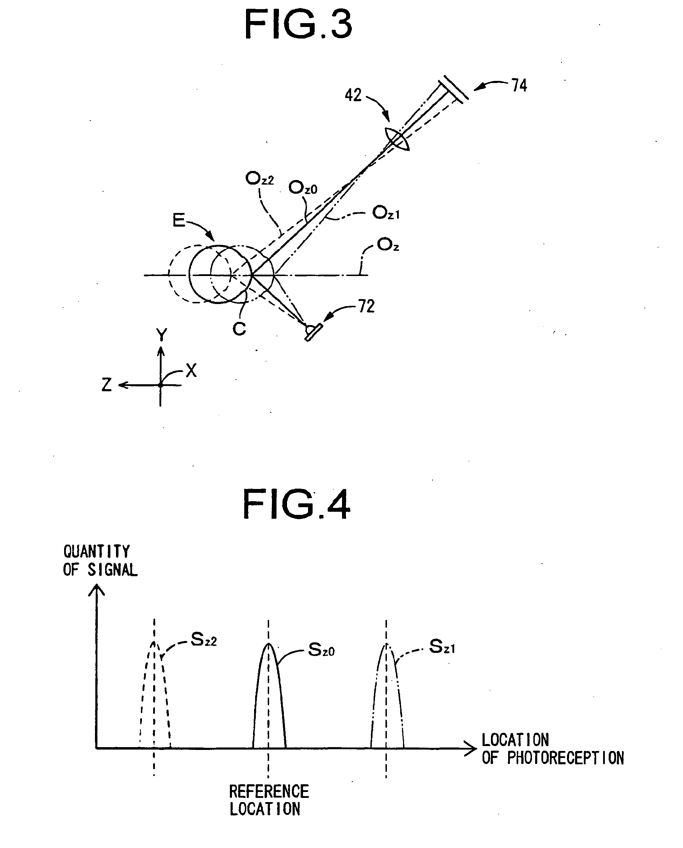

The Z alignment optical system 22 is composed of a Z alignment light source 72, the mirror 38, the mirror 40, the objective lens 42, the dichroic mirror 44, and a Z alignment sensor (profile sensor) 74 capable of sensing the position of the incident beam. The Z alignment light source 72 is a light source, such as an LED for example, having a different wavelength than that of the illuminant light source 24 of the illuminating optical system 12. In this embodiment in particular, the beam emitted by the Z alignment light source 72 and subsequently reflected by the examined eye E is infrared light so as not to impinge on the CCD 46 of the imaging optical system 14. The Z alignment sensor 74 is situated on an extension line of the optical axis O₂ that impinges on the dichroic mirror 44. In this way, the Z alignment optical system 22 shares in common with the imaging optical system 14 certain optical components, such as the mirrors 38, 40 and the objective lens 42 of the imaging optical system 14 so that the optical axis of the Z alignment optical system 22 is partially coincident with the optical axis O₂, of the imaging optical system 14. Additionally, the Z alignment optical system 22 is set up such that the optical axis along which light is directed onto the examined eye E from the Z alignment light source 72, and the optical axis along which light is reflected from the examined eye E and impinges on the mirror 38, are symmetrical with respect to the optical axis: O_{z} (see FIG. 3) of a measuring optical system, which is also the direction of a normal line passing through the apex of the cornea C at the location of completed alignment in the XY direction. In this embodiment in particular, the optical axis: O_{z0} of light reflected from the examined eye E will be coincident with the eye-side optical axis O₂ₑ of the imaging optical system 14 at the desired Z alignment location. The beam emitted by the Z alignment light source 72 and experiencing specular reflection at the cornea C of the examined eye E will be transmitted through the dichroic mirror 44 on the optical axis O₂ of the imaging optical system 14, and guided to the Z alignment sensor 74.

The apparatus optical system 10 described above is adapted to be displaceable in the X, Y, and Z directions by actuating means composed, for example, of a rack and pinion mechanism.

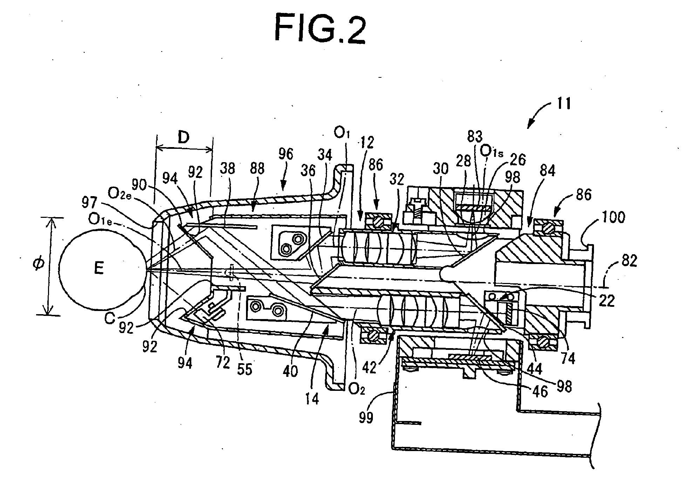

In this embodiment in particular, as depicted in model form in FIG. 2, the illuminating optical system 12 and the imaging optical system 14 are partially housed within a housing 84 that is rotatable about a center axis 82. FIG. 2 depicts in model form some of the constituent parts of an ocular anterior segment cross-sectional imaging apparatus 11 furnished with the apparatus optical system 10.

The housing 84 is generally cylindrical in shape extending with generally unchanging diameter dimension in the axial direction, and is rotatably supported at its axial ends about the center axis 82 by a pair of bearings 86. The center axis 82 which is the center of rotation of the housing 84 is identical to the eye-side optical axis O₁ₑ of the illuminating optical system 12. In this embodiment in particular, the housing 84 houses: the mirror 30; the projector lens 32; the dichroic mirror 36 that make up the illuminating optical system 12; the objective lens 42 and the dichroic mirror 44 that make up the imaging optical system 14; and the Z alignment sensor 74 that makes up the Z alignment optical system 22.

An inner cone 88 provided as an inner tube member is fixedly mounted at the axial end of the housing 84 on the examined eye E side. The inner cone 88 is generally cylindrical in shape and slightly smaller in diameter towards the examined eye E. The axial end on the opposite side from the examined eye E is open. At the axial end on the examined eye E side there is formed a screening wall 90 that makes it difficult to see into the interior of the inner cone 88 from the outside. Through-holes 92 are formed in appropriate areas of the screening wall 90 so as to avoid blocking the optical path of the apparatus optical system 10. In this embodiment in particular, projecting portions 94 that project towards the examined eye E are formed on the screening wall 90 in zones thereof situated to the outside of a pair of diametric lines that extend in the diametrical direction to either side of the center section of the inner cone 88 as viewed in the axial direction. By fixedly mounting the inner cone 88 so as to be positioned concentrically with the housing 84, the housing 84 and the inner cone 88 will be capable of rotation in unison about the center axis 82.

In this embodiment in particular, the inner cone 88 houses the mirror 34 that makes up the illuminating optical system 12; the mirror 38 and the mirror 40 that make up the imaging optical system 14; and the Z alignment light source 72 that makes up the Z alignment optical system 22. Of these, the mirror 38 that makes up the imaging optical system 14 and the Z alignment light source 72 are housed in the projecting portions 94. The mirror 38 and the Z alignment light source 72 can thus be situated closer to the examined eye E so that the reflected light beam from the examined eye E can be detected at a closer location, and the alignment beam can be directed in a diagonal direction from a location closer to the examined eye E. Further, the mirror 38 which is situated at the location closest to the examined eye E in the imaging optical system 14 is adapted to receive the reflected light beam from the examined eye E through one of the through-holes 92 formed in the screening wall 90. Thus, the outside peripheral rim (edge) of the mirror 38 will be concealed by the screening wall 90 and not readily visible from outside.

The inner cone 88 is positioned within an outer cone 96 as a cylindrical body. The outer cone 96 is generally cylindrical in shape and open at both axial ends, as well as being slightly smaller in diameter closer towards the examined eye E; at its axial end in proximity to the examined eye E the diameter dimension becomes sharply smaller. The aperture at the axial end closest to the examined eye E is a distal end aperture 97 provided as a front aperture. The diameter dimension (aperture dimension): φ of the distal end aperture 97 is set to a range from 20 mm to 60 mm, preferably from 30 mm to 50 mm. In this embodiment in particular, φ = 39 mm. If the diameter dimension: φ of the distal end aperture 97 is smaller than 20 mm, it will be difficult to position the illuminating optical system 12 and the imaging optical system 14 within the outer cone 96, whereas if larger than 60 mm, there is a risk that the outer cone 96 will bump against the patient's nose when attempting to position the outer cone 96 in proximity to the examined eye, so that it cannot be brought sufficiently close.

The outer cone 96 is fixedly disposed projecting out towards the examined eye E from the chassis section of the anterior segment cross-sectional imaging apparatus 11. In other words, the outer cone 96 is fixedly disposed at the location closest to the examined eye E within the anterior segment cross-sectional imaging apparatus 11; and encircles the entire circumference of the inner cone 88 in a non-contacting state, with a prescribed gap between them. Thus, the inner cone 88 will be able to rotate about the center axis 82 within the fixedly disposed outer cone 96, and the mirrors 34, 38, 40 etc. that are housed within the inner cone 88 will be able to rotate within the outer cone 96.

Thus, in this embodiment the illuminating optical system 12 and the imaging optical system 14 are both situated within the outer cone 96, and share the distal end aperture 97 of the outer cone 96 in common. Further, the optical axis between the mirror 30 and the mirror 34 in the illuminating optical system 12, and the optical axis between the mirror 40 and the dichroic mirror 44 in the imaging optical system 14, extend approximately parallel to the axial direction of the outer cone 96 (the left right direction in FIG. 2) within the outer cone 96, as viewed in the axial direction of the outer cone 96. The rotating housing 84 can thus be made more compact in size, and the housing 84 can be formed with generally unchanging diameter dimension without variation in the diameter dimension along the axial direction, so that production costs may be reduced as well. In this embodiment in particular, the reflecting mirrors that make up the illuminating optical system 12 and the imaging optical system 14, namely, the mirrors 30, 34, 38, 40 and the dichroic mirrors 36, 34, that is, substantially all of the reflecting mirrors that make up the illuminating optical system 12 and the imaging optical system 14, are housed within the outer cone 96 as viewed in the axial direction of the outer cone 96, and thus the rotating parts may be even more compact.

The separation distance: D from the distal end aperture 97 of the outer cone 96 to the mirror 38 (which initially reflects the scattered light from the examined eye E in the imaging optical system 14) in the direction of the eye-side optical axis O₁ₑ of the illuminating optical system 12 will be set to 40 mm or less, preferably to 35 mm or less. Namely, as there is an angular spread of prescribed inclination angle: α between the eye-side optical axis O₁ₑ of the illuminating optical system 12 and the eye-side optical axis O₂ₑ of the imaging optical system 14, if the installation location of the mirror 38 that deflects the direction of the eye-side optical axis O₂ₑ of the imaging optical system 14 is too far rearward, there is a risk that the device will be larger. The terminus point of the separation distance: D on the mirror 38 end is based on the location at which the eye-side optical axis O₂ₑ impinges on the mirror 38. In this embodiment in particular, the separation distance: D is set to 23.3 mm. However, the separation distance: D will be set appropriately in consideration of factors such as the aperture dimension: φ of the distal end aperture 97 of the outer cone 96, the number of reflecting mirrors, etc. For example, where the placement of the optical components of the illuminating optical system 12 and the imaging optical system 14 is the same as in the embodiment but the aperture dimension: φ of the distal end aperture 97 is different, by establishing appropriate values for separation distance: D based on a ratio to the aperture dimension: φ in the embodiment it will be possible to arrive at an appropriate value for separation distance: D of 17.9 mm in the case of 30 mm aperture dimension: φ (23.3 mm × 30/39), or an appropriate value for separation distance: D of 29.9 mm in the case of 50 mm aperture dimension: φ (23.3 mm × 50/39). However, no particular limitation is imposed by these values.

While not depicted in detail in the drawings, the slit 28, the projector lens 26, and the illuminant light source 24 which make up the illuminating optical system 12 and the CCD 46 which makes up the imaging optical system 14 are integrally disposed on the housing 84, to the outside of the housing 84. The peripheral wall of the housing 84 is perforated by through-holes 98 so that the optical paths of the illuminating optical system 12 and the imaging optical system 14 will not be obstructed. In this embodiment in particular, the illuminant light source 24 and the projector lens 26 will be arrayed in the direction perpendicular to the plane of the page in FIG. 2. A mirror 83 will be positioned at the point of intersection of the arrangement direction of the illuminant light source 24 and the projector lens 26 with the arrangement direction of the mirror 30 and the slit 28, to the outside of the housing 84. Thus, the beam exiting the illuminant light source 24 will be directed in the direction perpendicular to the plane of the page in FIG. 2, and after passing through the projector lens 26 will be deflected towards the housing 84 by the mirror 83 (downward in FIG. 2), passing through the slit 28 and striking the mirror 30. While not obvious from FIG. 2, a case 99 that houses the CCD 46 is fixedly disposed on the housing 84.

Through this arrangement, the illuminating optical system 12, the imaging optical system 14, and the Z alignment optical system 22 are capable of rotation in unison with the inner cone 88 and the housing 84 about the center axis 82, in other words, about the eye-side optical axis O₁ₑ of the illuminating optical system 12. While not depicted in detail in drawings, rotary actuating force will be transmitted to the housing 84 through an arrangement in which a grooved portion 100 formed at the axial back end of the housing 84 and opening axially outward is linked by a belt to the output shaft of a motor that provides rotary actuating force.

Meanwhile, while not depicted in the drawings, the observation optical system 16, the fixation target optical system 18, the XY alignment optical system 19, and the XY alignment detection optical system 20 are fixedly disposed to the rear of the housing 84 (the right side in FIG. 2). Consequently, in this embodiment, the illuminating optical system 12, the imaging optical system 14, and the Z alignment optical system 22 will rotate about the eye-side optical axis O₁ₑ of the illuminating optical system 12; while the observation optical system 16, the fixation target optical system 18, the XY alignment optical system 19, and the XY alignment detection optical system 20 will be fixedly disposed with their optical axes partially the same as the eye-side optical axis O₁ₑ of the illuminating optical system 12.

During cross-sectional imaging of the anterior ocular segment using the apparatus optical system 10 of the above construction, first, a fixation target light emitted by the fixation target light source 56 will pass through the fixation target optical system 18 and be guided into the examined eye E. The patient will then fix his or her gaze on the fixation target light so that the direction of the optical axis of the examined eye E is aligned with the optical axis O₃ of the observation optical system 16.

Furthermore, a beam emitted by the XY alignment light source 64 will be guided through the XY alignment optical system 19 and into the examined eye E. The beam reflected from the anterior segment of the examined eye E will then be guided into the XY alignment sensor 70 through the XY alignment detection optical system 20. The XY alignment sensor 70 has been designed to be able to sense the X direction location and the Y direction location of the beam that has been emitted by the XY alignment light source 64 and then reflected by the examined eye E. Then, on the basis of the sensed X direction location and Y direction location, alignment of the apparatus optical system 10 in the X direction and the Y direction (XY alignment) will be carried out by a drive mechanism (not shown) so as to bring the optical axis O₃ of the observation optical system 16 closer to the optical axis of the examined eye E.

Next, a beam is directed into the examined eye E from the Z alignment light source 72. The beam emitted by the Z alignment light source 72 will experience specular reflection in proximity to the apex of the cornea C of the examined eye E and be guided to the Z alignment sensor 74 through the Z alignment optical system 22. As depicted in model form in FIG. 3, as the examined eye E moves forward and backward in the Z direction relative to the Z alignment optical system 22, the location at which a reflected beam: O_{z1}, O_{z2} reflected from the examined eye E impinges on the Z alignment sensor 74 will shift from the impingement location of a reflected beam: O_{z0} reflected from the examined eye E when situated at an intended reference location. As a result, as depicted in model form in FIG. 4, the locations of photoreception signals S_{z1}, S_{z2} resulting sensing of the reflected beams: O_{z1}, O_{z2} at locations away from the intended location from the Z alignment sensor 74 will shift away from the location of the photoreception signal: S_{z0} resulting from sensing of the reflected beam: O_{z0} at the intended Z-direction location (i.e. the reference location at which offset = 0). Thus, the apparatus optical system 10 can be aligned in the Z direction (Z direction alignment) by driving it forward or backward in the Z direction using the drive mechanism (not shown), on the basis of the extent to which the photoreception signal sensed by the Z alignment sensor 74 is offset from the reference location.

Once alignment in the XY direction and the Z direction has been carried out in this way, a cross sectional image will be taken of the anterior segment of the examined eye E. First, a beam of light emitted by the illuminant light source 24 will be directed on the anterior face of the examined eye E through the illuminating optical system 12.

Then, the beam that was emitted by the illuminant light source 24 and scattered at the anterior segment of the examined eye E will be guided onto the CCD 46 through the imaging optical system 14. A cross sectional image of the anterior segment of the examined eye E will be taken in this way.

Next, the illuminating optical system 12 and the imaging optical system 14 will undergo prescribed angular rotation about the eye-side optical axis O₁ₑ of the illuminating optical system 12. Upon completion of the rotation, a cross sectional image will be taken of the anterior segment of the examined eye E. This rotation of the illuminating optical system 12 and the imaging optical system 14 and subsequent imaging will be carried out in prescribed angular increments around the entire circumference about the eye-side optical axis O₁ₑ of the illuminating optical system 12 so that cross sectional images are taken around the entire circumference of the anterior segment of the examined eye E. The rotation angle of the illuminating optical system 12 and the imaging optical system 14 may be either changing or unchanging around the entire circumference.

In the anterior segment cross-sectional imaging apparatus constructed in the above manner, by providing the imaging optical system 14 with the mirror 38 and the mirror 40, the direction of the beam scattered by the examined eye E will be brought closer to the direction of extension of the eye-side optical axis O₁ₑ of the illuminating optical system 12. Accordingly, the illuminating optical system 12 and the imaging optical system 14 can be formed at mutually proximate locations, making possible a more compact arrangement for the illuminating optical system 12 and the imaging optical system 14. In this embodiment in particular, the eye-side optical axis O₂ₑ of the imaging optical system 14 having a prescribed inclination angle: α with respect to the eye-side optical axis O₁ₑ of the illuminating optical system 12 will be brought closer to the optical axis O₁ₑ through reflection from the mirror 38. Moreover, having been brought into proximity with the optical axis O₁ₑ by the mirror 38, the optical axis O₂ₑ that has now passed the optical axis O₁ₑ and begun to again diverge from the optical axis O₁ₑ will be reflected by the mirror 40 and thereby again brought closer to the optical axis O₁ₑ, becoming approximately parallel to the direction of extension of the optical axis O₁ₑ. A more compact design will be possible thereby. In this way, in this embodiment the mirror 38 and the mirror 40 are included among the proximal direction reflecting mirrors that are used to reflect scattered light from the examined eye E in a direction in proximity to the optical axis of the illuminating optical system 12.

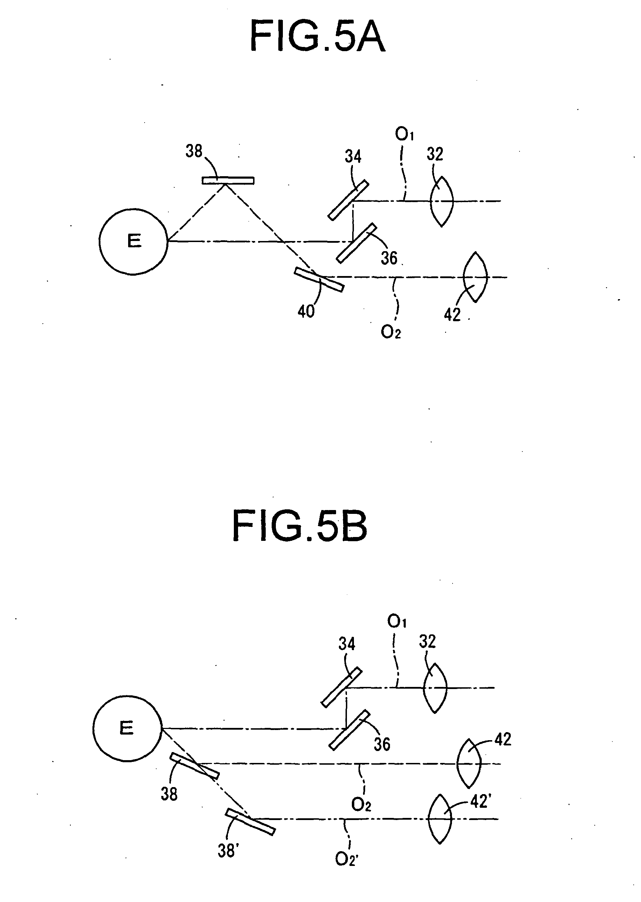

In this embodiment in particular, as depicted in FIG. 5A, better compactness can be achieved through the use of the two mirrors 38, 40. Specifically, if for example it is attempted to bring the optical axis: O₂ of the imaging optical system 14 into proximity with the optical axis: O₁, of the illuminating optical system 12 to the same extent as in this embodiment but using only a single mirror 38 as depicted in FIG. 5B, it will be necessary to position the mirror 38 quite close to the examined eye E, posing a risk that the mirror will contact the examined eye E or that the patient will experience discomfort. On the other hand, if a mirror 38' is positioned away from the examined eye E, the resultant optical axis: O₂' will be far away from the optical axis: O₁ of the illuminating optical system 12, incurring larger size of the apparatus. In contrast, through the use of two mirrors 38, 40 in accordance with this embodiment, the mirror 38 can be situated at a location appropriately far away from the examined eye E, while also bringing the optical axis: O₂ of the imaging optical system 14 into proximity with the optical axis: O₁ of the illuminating optical system 12 to achieve better compactness.

As a result of the greater compactness of the illuminating optical system 12 and the imaging optical system 14, it will be possible for the illuminating optical system 12 and the imaging optical system 14 to be accommodated within a common outer cone 96, with this outer cone 96 disposed projecting out from the chassis section of the imaging apparatus 11. Accordingly, the outer cone 96, and hence the illuminating optical system 12 and the imaging optical system 14, can be positioned in closer proximity to the examined eye E past the patient's nose. As a result, obstruction of the beam by the nose can be avoided, and localized missing areas of the image can be reduced. Additionally, infiltration of extraneous light into the illuminating optical system 12 and the imaging optical system 14 can be advantageously reduced, making it possible to carry out imaging in a lighted room without the need to use blackout curtains to block outside light, or to darken the room.

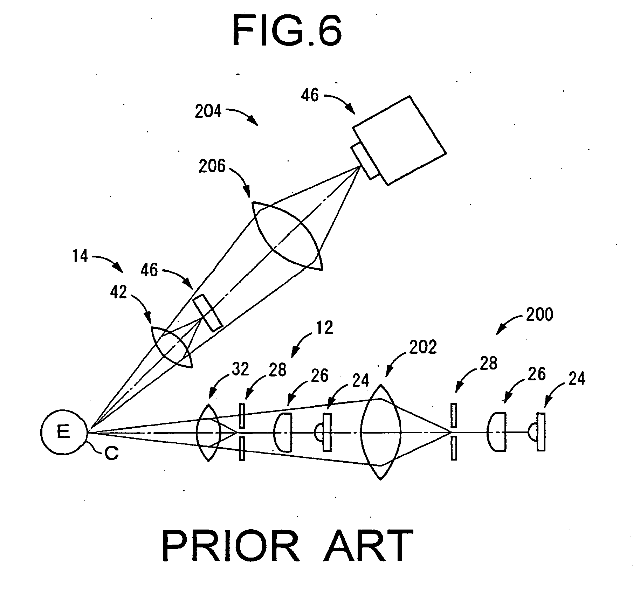

Also, as a result of being able to position the illuminating optical system 12 and the imaging optical system 14 in closer proximity to the examined eye E, the projector lens 32 and the objective lens 42 can be positioned at locations closer to the examined eye E. Thus, as compared with a projector lens 202 provided to an illuminating optical system 200 and an objective lens 206 provided to an imaging optical system 204 in a conventional design like that depicted in model form in FIG. 6, a sufficient quantity of light for imaging can be assured despite smaller lens diameter, thus affording further compactness. In FIG. 6, of the optical components that make up the illuminating optical system 200 and the imaging optical system 204 of conventional design, those other than projector lens 202 and the objective lens 206 have been assigned the same symbols as in the embodiment.

According to this embodiment, because it is possible for imaging to take place at a location closer to the examined eye E, the illuminating optical system 12 and the imaging optical system 14 will rotate at a location closer to the examined eye E; however, the optical systems 12, 14 are housed within the inner cone 88, with the inner cone 88 in turn being covered by the outer cone 96 which is fixed and does not rotate. Thus, safety will be ensured and it is unlikely that the patient will become conscious of the rotating illuminating optical system 12 and imaging optical system 14, so that the patient may be afforded a sense of assurance during the imaging process. In particular, according to this embodiment, because the outside peripheral rim (edge) of the mirror 38 which is positioned closest to the examined eye will be obscured by the screening wall 90 that has been formed on the inner cone 88, safety and assurance can be improved further.

Moreover, both the optical axis situated between the mirror 30 and the mirror 34 on the optical axis: O₁ of the illuminating optical system 12 and the optical axis situated between the mirror 40 and the dichroic mirror 44 on the optical axis: O₂ of the imaging optical system 14 extend approximately parallel to the shared optical axis: O₃ of the observation optical system 16 and the XY alignment optical system 19, at a location away from an extension line of the eye-side optical axis O₁ₑ of the illuminating optical system 12. Further compactness will be possible thereby. In particular, since these optical axes: O₁, O₂ are situated to either side of the optical axis: O₃ within the same plane, the illuminating optical system 12 and the imaging optical system 14 can be positioned to either side of the optical axis: O₃, and thus positioned with better weight balance about the optical axis: O₃ which represents the center of rotation. Sufficient installation space for the parts such as the projector lens 32 and the objective lens 42 that make up the illuminating optical system 12 and the imaging optical system 14 can be advantageously ensured as well.

Moreover, because a more compact arrangement is possible for the rotating illuminating optical system 12 and imaging optical system 14, rotation of these optical systems 12, 14 can take place faster. Thus, imaging of the entire circumference can be completed faster, diminished imaging accuracy due to tiny movements of the examined eye can will be reduced, and the burden on the patient can be reduced as well.

While the present invention has been described in detail hereinabove in terms of a preferred embodiment, the invention is in no way limited by the specific disclosure in the embodiment, and various changes, modifications, and improvements will be apparent to the skilled practitioner of the art. Such embodiment shall naturally be considered to fall within the scope of the present invention insofar as they do not depart from the spirit of the present invention.

For example, the apparatus optical system 10 shown above is merely exemplary, and installation locations for the lenses, slits etc. that make up each optical system are not limited to those hereinabove. For example, while in the preceding embodiment, the Z alignment optical system 22 is composed of mirrors 38, 40, etc. that are shared with the imaging optical system 14, it would also be acceptable for the Z alignment optical system 22 be independent of the imaging optical system 14. Where the Z alignment optical system 22 is constituted independently of the imaging optical system 14, the dichroic mirror 44 could be dispensed with, and the CCD 46 could be positioned at the location of the Z alignment sensor 74. Further, a CCD could be used in place of a profile sensor as the XY alignment sensor 70 or the Z alignment sensor 74.

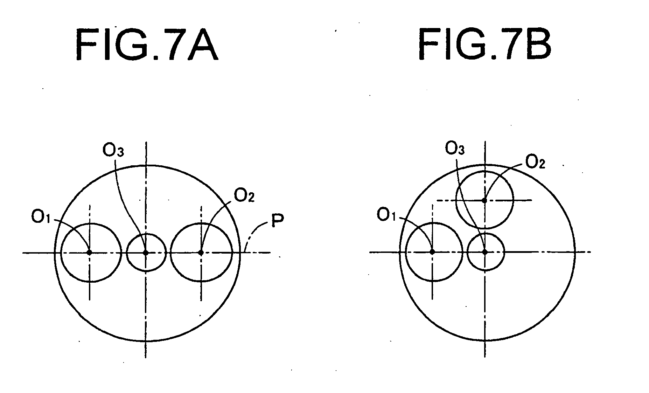

In the preceding embodiment, on the optical axis: O₁ of the illuminating optical system 12 and the optical axis: O₂ of the imaging optical system 14, the optical axes that extend in the same direction as the optical axis: O₃ of the observation optical system 16 (specifically, the optical axis between the mirror 30 and the mirror 34 on the optical axis: O₁ of the illuminating optical system 12, and the optical axis between the mirror 40 and the dichroic mirror 44 on the optical axis: O₂ of the imaging optical system 14) are situated to either side of the optical axis: O₃ of the observation optical system 16 within the same plane: P viewed in the axial direction of the outer cone 96 as depicted in FIG. 7A. Alternatively, they could instead be situated in different planes, as depicted in FIG. 7B.

Furthermore, the specific shape and dimensions of the cylindrical body and inner tube member housing the illuminating optical system and the imaging optical system are not particularly limited to those shown above by way of example. The outer cone 96 in the above embodiment may be given tubular shape of unchanging diameter dimension in the axial direction; and the projecting portions 94 of the inner cone 88 are not an essential element.

It is sufficient for at least one reflecting mirror to be disposed on the optical axis of the imaging optical system for the purpose of reflecting scattered light from the examined eye to a direction closer to the optical axis of the illuminating optical system, and the number thereof is not limited in any way. For example, it would be possible to provide an additional mirror or mirrors in addition to the mirrors 38, 40 shown in the embodiment. Additionally, it is not necessary for all of the reflecting mirrors to be situated inside the inner tube member; for example, the mirror 40 of the embodiment could instead be housed within the housing 84.

## Claims

1. An apparatus for cross-sectional imaging of an anterior ocular segment (10), comprising:
an illuminating optical system (12) furnished with an illuminating light source (24) for directing a slit beam from a front onto an examined eye (E);
an imaging optical system (14) furnished with a photoreceptor element (46) that has an eye-side optical axis (O₂ₑ) inclined by a prescribed angle (α) with respect to an eye-side optical axis (O₁ₑ) of the illuminating optical system (12) and that detects scattered light produced by scattering of the slit beam by the examined eye (E) and images a cross section of the examined eye (E); **characterised in that** the apparatus further comprises:
a stationary cylindrical body (96) having apertures at both axial ends and being disposed projecting out from a body of the apparatus;
the illuminating optical system (12) and the imaging optical system (14) being disposed within the cylindrical body (96) with a front aperture (97) of the cylindrical body (96) as a common aperture thereof so as to be rotatable in unison about the eye-side optical axis (O₁ₑ) of the illuminating optical system (12); and
at least one proximal direction reflecting mirror (38, 40) being disposed within an interior of the cylindrical body (96) in proximity to the front aperture for reflecting scattered light by the examined eye (E) on an optical axis (O₂) of the imaging optical system (14) to a direction proximate to an optical axis (O₁) of the illuminating optical system (12).

2. The apparatus for cross-sectional imaging of an anterior ocular segment (10) according to claim 1, wherein the front aperture (97) of the cylindrical body (96) has a diameter dimension held within a range from 20 mm to 60 mm.

3. The apparatus for cross-sectional imaging of an anterior ocular segment (10) according to claim 1 or 2, wherein the proximal direction reflecting mirror (38) for reflecting the eye-side optical axis (O₂ₑ) of the imaging optical system (14) is disposed at location 40 mm or less from the front aperture (97) of the cylindrical body (96) in the direction of the eye-side optical axis (O₁ₑ) of the illuminating optical system (12).

4. The apparatus for cross-sectional imaging of an anterior ocular segment (10) according to any one of claims 1-3, wherein an output optical axis (O₁ₛ) of the illuminating optical system (12) is orthogonal to the eye-side optical axis (O₁ₑ) of the illuminating optical system (12).

5. The apparatus for cross-sectional imaging of an anterior ocular segment (10) according to any one of claims 1-4, wherein a photoreception face of the photoreceptor element (46) of the imaging optical system (14) is oriented parallel to the eye-side optical axis (O₁ₑ) of the illuminating optical system (12).

6. The apparatus for cross-sectional imaging of an anterior ocular segment (10) according to any one of claims 1-5, wherein the illuminating optical system (12), the imaging optical system (14), and the proximal direction reflecting mirror (38, 40) are situated inside an inner tube member (88) that has been disposed inside the cylindrical body (96) such that the illuminating optical system (12), the imaging optical system (14), and the proximal direction reflecting mirror (38, 40) are capable of rotation in unison with the inner tube member (88) about the eye-side optical axis (O₁ₑ) of the illuminating optical system (12); and the inner tube member (88) is provided with a screening wall (90) whereby an outside peripheral rim of the proximal direction reflecting mirror (38, 40) is not readily visible from an outside.

7. The apparatus for cross-sectional imaging of an anterior ocular segment (10) according to any one of claims 1-6, wherein the at least one proximal direction reflecting mirror comprises a plurality of proximal direction reflecting mirrors (38, 40) that are all situated inside the cylindrical body (96).

8. The apparatus for cross-sectional imaging of an anterior ocular segment (10) according to any one of claims 1-7, wherein the at least one proximal direction reflecting mirror comprises a plurality of proximal direction reflecting mirrors (30, 34, 38, 40) that make up the illuminating optical system (12) and the imaging optical system (14) are situated within the cylindrical body (96) as viewed in an axial direction of the cylindrical body (96); and the optical axis (O₁) of the illuminating optical system (12) and the optical axis (O₂) of the imaging optical system (14) extend in the axial direction of the cylindrical body (96) within the cylindrical body (96) as viewed in the axial direction of the cylindrical body (96).

9. The apparatus for cross-sectional imaging of an anterior ocular segment (10) according to any one of claims 1-8, further comprising XY direction position sensing means (19) for sensing relative optical position of the illuminating optical system (12) and the imaging optical system (14) with respect to the examined eye (E) in XY directions orthogonal to a Z direction which is a direction of moving closer to and away from the examined eye (E), as the illuminating optical system (12) and the imaging optical system (14) are moved in the XY directions, wherein the XY direction position sensing means (19) is fixedly disposed such that an optical axis thereof and the eye-side optical axis (O₁ₑ) of the illuminating optical system (12) are identical.

10. The apparatus for cross-sectional imaging of an anterior ocular segment (10) according to claim 9, wherein the optical axis (O₁) of the illuminating optical system (12) and the optical axis (O₂) of the imaging optical system (14) extend in a same direction as an optical axis (O₃) of the XY direction position sensing means (19) at a location away from the eye-side optical axis (O₁ₑ) of the illuminating optical system (12); and the optical axes (O₁, O₂) of the illuminating optical system (12) and the imaging optical system (14) that extend in the same direction as the optical axis (O₃) of the XY direction position sensing means (19) are positioned at different circumferential locations about the optical axis (O₃) of the XY direction position sensing means (19).

11. The apparatus for cross-sectional imaging of an anterior ocular segment (10) according to any one of claims 1-10, further comprising: Z direction position sensing means (22) for sensing relative optical positions of the illuminating optical system (12) and the imaging optical system (14) in the Z direction with respect to the examined eye (E) and adapted to rotate in unison with the illuminating optical system (12) and the imaging optical system (14) during movement of the illuminating optical system (12) and the imaging optical system (14) in the Z direction, which is the direction of moving closer to and away from the examined eye (E).

## Patentansprüche

1. Vorrichtung zur Querschnittsabbildung eines vorderen Augensegments (10), die umfasst:
ein optisches Beleuchtungssystem (12), das mit einer Beleuchtungslichtquelle (24) zum Richten eines Spaltstrahls von einer Vorderseite auf ein untersuchtes Auge (E) versehen ist;
ein optisches Abbildungssystem (14), das mit einem Photorezeptorelement (46) versehen ist, das eine augenseitige optische Achse (O₂ₑ) aufweist, die um einen vorgeschriebenen Winkel (α) in Bezug auf eine augenseitige optische Achse (O₁ₑ) des optischen Beleuchtungssystems (12) geneigt ist, und das gestreutes Licht detektiert, das durch Streuen des Spaltstrahls durch das untersuchte Auge (E) erzeugt wird, und einen Querschnitt des untersuchten Auges (E) abbildet; **dadurch gekennzeichnet, dass** die Vorrichtung ferner umfasst:
einen stationären zylindrischen Körper (96) mit Öffnungen an beiden axialen Enden, der so angeordnet ist, dass er aus einem Körper der Vorrichtung vorsteht;
wobei das optische Beleuchtungssystem (12) und das optische Abbildungs-system (14) innerhalb des zylindrischen Körpers (96) mit einer vorderen Öffnung (97) des zylindrischen Körpers (96) als gemeinsamer Öffnung davon so angeordnet sind, dass sie gemeinsam um die augenseitige optische Achse (O₁ₑ) des optischen Beleuchtungssystems (12) drehbar sind; und
wobei mindestens ein proximaler Richtungsreflexionsspiegel (38, 40) in einem Inneren des zylindrischen Körpers (96) in der Nähe der vorderen Öffnung angeordnet ist, um durch das untersuchte Auge (E) gestreutes Licht auf einer optischen Achse (O₂) des optischen Abbildungssystems (14) in eine Richtung nahe einer optischen Achse (O₁) des optischen Beleuchtungssystems (12) zu reflektieren.

2. Vorrichtung zur Querschnittsabbildung eines vorderen Augensegments (10) nach Anspruch 1, wobei die vordere Öffnung (97) des zylindrischen Körpers (96) eine Durchmesserabmessung aufweist, die innerhalb eines Bereichs von 20 mm bis 60 mm gehalten wird.

3. Vorrichtung zur Querschnittsabbildung eines vorderen Augensegments (10) nach Anspruch 1 oder 2, wobei der proximale Richtungsreflexionsspiegel (38) zum Reflektieren der augenseitigen optischen Achse (O₂ₑ) des optischen Abbildungs-systems (14) an einem Ort 40 mm oder weniger von der vorderen Öffnung (97) des zylindrischen Körpers (96) in der Richtung der augenseitigen optischen Achse (O₁ₑ) des optischen Beleuchtungssystems (12) angeordnet ist.

4. Vorrichtung zur Querschnittsabbildung eines vorderen Augensegments (10) nach einem der Ansprüche 1-3, wobei eine optische Ausgangsachse (O₁ₛ) des optischen Beleuchtungssystems (12) zur augenseitigen optischen Achse (O₁ₑ) des optischen Beleuchtungssystems (12) senkrecht ist.

5. Vorrichtung zur Querschnittsabbildung eines vorderen Augensegments (10) nach einem der Ansprüche 1-4, wobei eine Photorezeptionsfläche des Photorezeptorelements (46) des optischen Abbildungssystems (14) parallel zur augenseitigen optischen Achse (O₁ₑ) des optischen Beleuchtungssystems orientiert ist.

6. Vorrichtung zur Querschnittsabbildung eines vorderen Augensegments (10) nach einem der Ansprüche 1-5, wobei das optische Beleuchtungssystem (12), das optische Abbildungssystem (14) und der proximale Richtungsreflexionsspiegel (38, 40) innerhalb eines inneren Rohrelements (88) liegen, das innerhalb des zylindrischen Körpers (96) angeordnet wurde, so dass das optische Beleuchtungssystem (12), das optische Abbildungssystem (14) und der proximale Richtungsreflexionsspiegel (38, 40) zu einer gemeinsamen Drehung mit dem inneren Rohrelement (88) um die augenseitige optische Achse (O₁ₑ) des optischen Beleuchtungssystems (12) in der Lage sind; und das innere Rohrelement (88) mit einer Abschirmwand (90) versehen ist, wodurch ein äußerer Umfangsrand des proximalen Richtungsreflexionsspiegels (38, 40) von einer Außenseite nicht leicht sichtbar ist.

7. Vorrichtung zur Querschnittsabbildung eines vorderen Augensegments (10) nach einem der Ansprüche 1-6, wobei der mindestens eine proximale Richtungsreflexionsspiegel mehrere proximale Richtungsreflexionsspiegel (38, 40) umfasst, die alle innerhalb des zylindrischen Körpers (96) liegen.

8. Vorrichtung zur Querschnittsabbildung eines vorderen Augensegments (10) nach einem der Ansprüche 1-7, wobei der mindestens eine proximale Richtungreflexionsspiegel mehrere proximale Richtungsreflexionsspiegel (30, 34, 38, 40) umfasst, die das optische Beleuchtungssystem (12) und das optische Abbildungs-system (14) bilden und in einer axialen Richtung des zylindrischen Körpers (96) betrachtet innerhalb des zylindrischen Körpers (96) liegen; und die optische Achse (O₁) des optischen Beleuchtungssystems (12) und die optische Achse (O₂) des optischen Abbildungssystems (14) in der axialen Richtung des zylindrischen Körpers (96) betrachtet sich in der axialen Richtung des zylindrischen Körpers (96) innerhalb des zylindrischen Körpers (96) erstrecken.

9. Vorrichtung zur Querschnittsabbildung eines vorderen Augensegments (10) nach einem der Ansprüche 1-8, die ferner ein XY-Richtungs-Positionserfassungsmittel (19) zum Erfassen der relativen optischen Position des optischen Beleuchtungssystems (12) und des optischen Abbildungssystems (14) in Bezug auf das untersuchte Auge (E) in den XY-Richtungen umfasst, die zu einer Z-Richtung senkrecht sind, die eine Richtung zum Bewegen näher zum untersuchten Auge (E) und von diesem weg ist, wenn das optische Beleuchtungssystem (12) und das optische Abbildungssystem (14) in den XY-Richtungen bewegt werden, wobei das XY-Richtungs-Positionserfassungsmittel (19) fest angeordnet ist, so dass eine optische Achse davon und die augenseitige optische Achse (O₁ₑ) des optischen Beleuchtungssystems (12) identisch sind.

10. Vorrichtung zur Querschnittsabbildung eines vorderen Augensegments (10) nach Anspruch 9, wobei sich die optische Achse (O₁) des optischen Beleuchtungssystems (12) und die optische Achse (O₂) des optischen Abbildungssystems (14) an einer Stelle, die von der augenseitigen optischen Achse (O₁ₑ) des optischen Beleuchtungssystems (12) entfernt ist, in einer gleichen Richtung wie eine optische Achse (O₃) des XY-Richtungs-Positionserfassungsmittels (19) erstrecken; und die optischen Achsen (O₁, O₂) des optischen Beleuchtungssystems (12) und des optischen Abbildungssystems (14), die sich in derselben Richtung wie die optische Achse (O₃) des XY-Richtungs-Positionserfassungsmittels (19) erstrecken, an verschiedenen Umfangsstellen um die optische Achse (O₃) des XY-Richtungs-Positionserfassungsmittels (19) angeordnet sind.

11. Vorrichtung zur Querschnittsabbildung eines vorderen Augensegments (10) nach einem der Ansprüche 1-10, die ferner umfasst: ein Z-Richtungs-Positionsertassungsmittel (22) zum Erfassen von relativen optischen Positionen des optischen Beleuchtungssystems (12) und des optischen Abbildungssystems (14) in der Z-Richtung in Bezug auf das untersuchte Auge (E), das dazu ausgelegt ist, sich gemeinsam mit dem optischen Beleuchtungssystem (12) und dem optischen Abbildungssystem (14) während der Bewegung des optischen Beleuchtungssystems (12) und des optischen Abbildungssystems (14) in der Z-Richtung zu drehen, welche die Richtung zum Bewegen näher zum untersuchten Auge (E) und von diesem weg ist.

## Revendications

1. Dispositif pour l'imagerie en coupe d'un segment oculaire antérieur (10), comprenant :
un système optique d'éclairage (12) pourvu d'une source de lumière d'éclairage (24) pour diriger un faisceau de fente depuis une partie avant sur un oeil examiné (E) ;
un système optique d'imagerie (14) pourvu d'un élément photorécepteur (46) qui a un axe optique côté oeil (O₂ₑ) incliné d'un angle prescrit (α) par rapport à un axe optique côté oeil (O₁ₑ) du système optique d'éclairage (12) et qui détecte la lumière diffusée produite par la diffusion du faisceau de fente réalisée par l'oeil examiné (E) et prend une image d'une coupe de l'oeil examiné (E) ;
**caractérisé en ce que** le dispositif comprend en outre :
un corps cylindrique statique (96) comportant des ouvertures à ses deux extrémités axiales et étant positionné de façon à faire saillie d'un corps du dispositif ;
le système optique d'éclairage (12) et le système optique d'imagerie (14) étant placés à l'intérieur du corps cylindrique (96) avec une ouverture avant (97) du corps cylindrique (96) comme ouverture commune de ceux-ci afin qu'ils puissent tourner à l'unisson autour de l'axe optique côté oeil (O₁ₑ) du système optique d'éclairage (12) ; et
au moins un miroir réfléchissant dans une direction proximale (38, 40) étant placé à l'intérieur du corps cylindrique (96) à proximité de l'ouverture avant pour réfléchir la lumière diffusée par l'oeil examiné (E) sur un axe optique (O₂) du système optique d'imagerie (14) vers une direction proche d'un axe optique (O₁) du système optique d'éclairage (12).

2. Dispositif pour l'imagerie en coupe d'un segment oculaire antérieur (10) selon la revendication 1, dans lequel l'ouverture avant (97) du corps cylindrique (96) a une dimension de diamètre maintenue dans un intervalle de 20 mm à 60 mm.

3. Dispositif pour l'imagerie en coupe d'un segment oculaire antérieur (10) selon la revendication 1 ou 2, dans lequel le miroir réfléchissant dans une direction proximale (38) pour réfléchir l'axe optique côté oeil (O₂ₑ) du système optique d'imagerie (14) est placé à un emplacement situé à 40 mm ou moins de l'ouverture avant (97) du corps cylindrique (96) dans la direction de l'axe optique côté oeil (O₁ₑ) du système optique d'éclairage (12).

4. Dispositif pour l'imagerie en coupe d'un segment oculaire antérieur (10) selon l'une quelconque des revendications 1 à 3, dans lequel un axe optique de sortie (O₁ₛ) du système optique d'éclairage (12) est orthogonal à l'axe optique côté oeil (O₁ₑ) du système optique d'éclairage (12).

5. Dispositif pour l'imagerie en coupe d'un segment oculaire antérieur (10) selon l'une quelconque des revendications 1 à 4, dans lequel une face de photoréception de l'élément photorécepteur (46) du système optique d'imagerie (14) est orientée parallèlement à l'axe optique côté oeil (O₁ₑ) du système optique d'éclairage (12).

6. Dispositif pour l'imagerie en coupe d'un segment oculaire antérieur (10) selon l'une quelconque des revendications 1 à 5, dans lequel le système optique d'éclairage (12), le système optique d'imagerie (14) et le miroir réfléchissant dans une direction proximale (38, 40) se trouvent à l'intérieur d'un élément formant tube intérieur (88) qui a été placé à l'intérieur du corps cylindrique (96) de telle manière que le système optique d'éclairage (12), le système optique d'imagerie (14) et le miroir réfléchissant dans une direction proximale (38, 40) sont aptes à tourner à l'unisson avec l'élément formant tube intérieur (88) autour de l'axe optique côté oeil (O₁ₑ) du système optique d'éclairage (12), et l'élément formant tube intérieur (88) est pourvu d'une paroi formant écran (90) grâce à laquelle un rebord périphérique extérieur du miroir réfléchissant dans une direction proximale (38, 40) n'est pas facilement visible de l'extérieur.

7. Dispositif pour l'imagerie en coupe d'un segment oculaire antérieur (10) selon l'une quelconque des revendications 1 à 6, dans lequel ledit au moins un miroir réfléchissant dans une direction proximale comprend une pluralité de miroirs réfléchissant dans une direction proximale (38, 40) qui sont tous situés à l'intérieur du corps cylindrique (96).

8. Dispositif pour l'imagerie en coupe d'un segment oculaire antérieur (10) selon l'une quelconque des revendications 1 à 7, dans lequel ledit au moins un miroir réfléchissant dans une direction proximale comprend une pluralité de miroirs réfléchissant dans une direction proximale (30, 34, 38, 40) qui constituent le système optique d'éclairage (12) et le système optique d'imagerie (14) et qui sont situés à l'intérieur du corps cylindrique (96), vus dans une direction axiale du corps cylindrique (96), et l'axe optique (O₁) du système optique d'éclairage (12) et l'axe optique (O₂) du système optique d'imagerie (14) s'étendent dans la direction axiale du corps cylindrique (96) à l'intérieur du corps cylindrique (96) vu dans la direction axiale du corps cylindrique (96).

9. Dispositif pour l'imagerie en coupe d'un segment oculaire antérieur (10) selon l'une quelconque des revendications 1 à 8, comprenant en outre un moyen de mesure de position dans des directions XY (19) pour mesurer la position optique relative du système optique d'éclairage (12) et du système optique d'imagerie (14) par rapport à l'oeil examiné (E) dans des directions XY orthogonales à une direction Z qui est une direction dans laquelle on s'approche et on s'éloigne de l'oeil examiné (E), pendant que le système optique d'éclairage (12) et le système optique d'imagerie (14) sont déplacés dans les directions XY, le moyen de mesure de position dans des directions XY (19) étant disposé de manière fixe, de telle manière qu'un axe optique de celui-ci et l'axe optique côté oeil (O₁ₑ) du système optique d'éclairage (12) sont identiques.

10. Dispositif pour l'imagerie en coupe d'un segment oculaire antérieur (10) selon la revendication 9, dans lequel l'axe optique (O₁) du système optique d'éclairage (12) et l'axe optique (O₂) du système optique d'imagerie (14) s'étendent dans une même direction qu'un axe optique (O₃) du moyen de mesure de position dans des directions XY (19) en un emplacement éloigné de l'axe optique côté oeil (O₁ₑ) du système optique d'éclairage (12), et les axes optiques (O₁, O₂) du système optique d'éclairage (12) et du système optique d'imagerie (14) qui s'étendent dans la même direction que l'axe optique (O₃) du moyen de mesure de position dans des directions XY (19) sont positionnés en différents emplacements circonférentiels autour de l'axe optique (O₃) du moyen de mesure de position dans des directions XY (19).

11. Dispositif pour l'imagerie en coupe d'un segment oculaire antérieur (10) selon l'une quelconque des revendications 1 à 10, comprenant en outre un moyen de mesure de position dans une direction Z (22) pour mesurer les positions optiques relatives du système optique d'éclairage (12) et du système optique d'imagerie (14) dans la direction Z par rapport à l'oeil examiné (E) et adapté pour tourner à l'unisson avec le système optique d'éclairage (12) et le système optique d'imagerie (14) pendant le mouvement du système optique d'éclairage (12) et du système optique d'imagerie (14) dans la direction Z, qui est direction dans laquelle on s'approche et on s'éloigne de l'oeil examiné (E).
